# EUROPEAN PATENT SPECIFICATION

(11) **EP 2 945 637 B1**
(45) Date of publication and mention of the grant of the patent: **10.03.2021**
(21) Application number: 14703425.0
(22) Date of filing: 13.01.2014
(51) Int. Cl.: A61K 31/55, A61K 31/506, A61K 31/4545, A61K 31/501, A61P 43/00, A61P 25/00, A61P 25/26, A61P 31/00, A61P 31/18

(54) **USE OF ALPHA 7 NICOTINIC ACETYLCHOLINE RECEPTOR AGONISTS**
VERWENDUNG VON ALPHA-7-NIKOTINISCHEN ACETYLCHOLINREZEPTORAGONISTEN
UTILISATION D'AGONISTES DU RECEPTEUR NICOTINIQUE DE L' ACETYLCHOLINE ALPHA 7

(30) Priority: 15.01.2013 US 201361752765 P
(43) Date of publication of application: 25.11.2015
(73) Proprietor: Novartis AG, 4056 Basel (CH)
(72) Inventor: FENDT, Markus, CH-4002 Basel (CH); FEUERBACH, Dominik, CH-4002 Basel (CH); JOHNS, Donald, Cambridge, Massachusetts 02139 (US); LOPEZ-LOPEZ, Cristina, CH-4002 Basel (CH); MCALLISTER, Kevin Hall, CH-4002 Basel (CH); SOVAGO, Judit, CH-4002 Basel (CH); WEISS, Markus, CH-4002 Basel (CH)
(74) Representative: Appleyard Lees IP LLP
(86) International application number: PCT/IB2014/058224
(87) International publication number: WO 2014/111837

(56) References cited:
- WO-A1-01/85727
- WO-A1-2004/016608
- WO-A1-2004/022556
- WO-A1-2005/123732
- WO-A1-2006/005608
- WO-A1-2007/045478
- WO-A1-2007/068475
- WO-A1-2007/068476
- WO-A1-2007/093602
- WO-A2-2011/009890
- DANIELA GÜNDISCH ET AL: "Nicotinic acetylcholine receptor ligands, a patent review (2006 - 2011)", EXPERT OPINION ON THERAPEUTIC PATENTS, vol. 21, no. 12, 1 December 2011 (2011-12-01), pages 1867-1896, XP055067779, ISSN: 1354-3776, DOI: 10.1517/13543776.2011.637919
- ANATOLY A. MAZUROV ET AL: "Discovery and Development of [alpha]7 Nicotinic Acetylcholine Receptor Modulators", JOURNAL OF MEDICINAL CHEMISTRY, vol. 54, no. 23, 15 September 2011 (2011-09-15), pages 7943-7961, XP055060345, ISSN: 0022-2623, DOI: 10.1021/jm2007672
- AFARI N ET AL: "CHRONIC FATIGUE SYNDROME: A REVIEW", AMERICAN JOURNAL OF PSYCHIATRY, AMERICAN PSYCHIATRIC PUBLISHING, INC, US, vol. 160, no. 2, 1 February 2003 (2003-02-01), pages 221-236, XP008072634, ISSN: 0002-953X, DOI: 10.1176/APPI.AJP.160.2.221
- ARIAS H R ET AL: "Different interaction between the agonist JN403 and the competitive antagonist methyllycaconitine with the human alpha7 nicotinic acetylcholine receptor", BIOCHEMISTRY, AMERICAN CHEMICAL SOCIETY, US, vol. 49, no. 19, 18 May 2010 (2010-05-18), pages 4169-4180, XP002694125, ISSN: 0006-2960, DOI: 10.1021/BI901999V [retrieved on 2010-04-09]
- FEUERBACH D ET AL: "The selective nicotinic acetylcholine receptor alpha7 agonist JN403 is active in animal models of cognition, sensory gating, epilepsy and pain", NEUROPHARMACOLOGY, PERGAMON PRESS, OXFORD, GB, vol. 56, no. 1, 1 January 2009 (2009-01-01), pages 254-263, XP025846225, ISSN: 0028-3908, DOI: 10.1016/J.NEUROPHARM.2008.08.025 [retrieved on 2008-08-28]

## Description

The present invention relates to pharmaceutical uses of alpha 7 nicotinic acetylcholine receptor (α7 nAChR) agonists.

Fatigue is a prolonged state in a subject which is characterized by physical and/or mental weakness and is caused by a disorder.

Said physical weakness is the temporary physical inability of a muscle to perform optimally. It may also be described as muscle exhaustion, muscle weakness, muscle ache or lack of strength and relates to the inability to exert force with one's muscles to the degree that would be expected given the individual's general physical fitness. Central muscle weakness is an overall exhaustion of the whole body, while peripheral weakness is an exhaustion of individual muscles.

Mental weakness is a temporary inability to maintain optimal cognitive performance. It is characterized by lack of motivation and of alertness. The onset during any cognitive activity is gradual and depends upon an individual's cognitive ability and also upon other factors, such as sleep deprivation and overall health. Mental weakness can manifest itself as sleepiness (decreased wakefulness) or as a general decrease of attention, not necessarily including sleepiness. Decreased attention is also known as "ego depletion" and may be described as a more or less decreased level of consciousness. This may be dangerous when performing tasks that require constant concentration, e.g. driving a vehicle.

Although similar, fatigue-like states in subjects may be the result of working, mental stress, overstimulation, understimulation, jet lag, boredom and lack of sleep; said fatigue-like states are not caused by a disorder and are usually reversed by a night's sleep.

Fatigue can be e.g. chronic fatigue syndrome (CFS).
Fatigue can be caused by any of the following disorders: autoimmune diseases such as multiple sclerosis (MS), celiac disease and spondyloarthropathy; infectious diseases, such as HIV infection or infectious mononucleosis; blood disorders, such as anemia and hemochromatosis; cancer (in which case it is also called cancer fatigue); drug abuse, including alcohol abuse; depression or other mental disorders that feature depressed mood; eating disorders (which can produce fatigue due to inadequate nutrition); endocrine diseases, e.g. diabetes mellitus and hypothyroidism; fibromyalgia; Gulf War Syndrome; heart disease; irritable bowel syndrome; inborn errors of metabolism, e.g. fructose malabsorption; liver failure; leukemia; lymphoma; Lyme disease; neurological disorders, e.g. Parkinson's disease and post-concussion syndrome; physical trauma and other pain-causing conditions, such as arthritis; uremia which is caused by kidney disease; and stroke. Fatigue can also be indirectly caused by a disorder, e.g. can be a side effect of medications used to treat a disorder, e.g. lithium salts; ciprofloxacin; beta blockers, which can induce exercise intolerance; and cancer treatments, e.g. chemotherapy and radiotherapy.

WO2005123732 describes 1-aza-bicyclo[3.3.1]nonanes as a pharmaceutical for the prevention and the treatment of psychotic and neurodegenerative disorders.

WO2004022556 describes aza-bicycloalkyl ethers which are alpha 7 nicotinic acetylcholine receptor (nAChR) agonists.

"Chronic Fatigue Syndrome: A Review", Afari, N, American Journal of Psychiatry, vol. 160, no. 2, pages 221 - 236 concludes that chronic fatigue syndrome is unlikely to be caused or maintained by a single agent and that assessment and treatment of should be multidimensional and tailored to the needs of the individual patient.

WO2011009890 describes the use of azabicydoalkyl derivatives or pyrroiidine-2-one derivatives for the treatment, prevention or delay of progression of ataxia.

There is a need for new pharmacological compounds that effectively target fatigue.

It has been found that certain α7 nAChR agonists may be used in the treatment (whether therapeutic or prophylactic), prevention, amelioration or delay of progression of fatigue.

Accordingly, a first aspect of the invention concerns an alpha 7 nicotinic acetylcholine receptor agonist for use in the treatment, amelioration, prevention or delay of progression of fatigue caused by a disorder;
wherein said alpha 7 nicotinic acetylcholine receptor agonist is (R)-3-(6-p-tolyl-pyridin-3-yloxy)-1-aza-bicyclo[2.2.2]octane in free base form or in acid addition salt form.

Disclosed herein is a method for the treatment, amelioration, prevention or delay of progression of fatigue in a subject in need of such treatment, which comprises administering to said subject a therapeutically effective amount of the α7 nAChR agonist of the invention.

Disclosed herein is the use of the α7 nAChR agonist of the invention for the manufacture of a medicament for the treatment, amelioration, prevention or delay of progression of fatigue.

### α7 nAChR agonist of the invention:

As used herein an "α7 nAChR agonist" is a compound that binds to a receptor comprising an α7 nAChR subunit in vivo and in vitro and is activating the receptor. Activation can be measured by the method disclosed in WO2001/85727, i.e. a functional affinity assay at the homomeric α7 nAChR carried out with a rat pituitary cell line stably expressing the α7 nAChR. As read out, the calcium influx upon stimulation of the receptor compared to epibatidine is used. "α7 nAChR agonists of the invention" according to the invention typically induce calcium influx of at least 50% of the maximal influx evoked by epibatidine with an EC₅₀ value of at least 1µM.

In one embodiment, the α7 nAChR agonist of the invention is selective for a receptor comprising a nicotinic acetylcholine receptor alpha 7 subunit, since such an agonist would be expected to cause fewer side effects than a non-selective agonist to a treated subject. An agonist being selective for a receptor comprising a nicotinic acetylcholine receptor alpha 7 subunit has a functional affinity to such a receptor to a much higher degree, e.g. at least 10-fold affinity difference in EC₅₀ value, preferably at least 20-fold, more preferably at least 50-fold, compared to any other nicotinic acetylcholine receptor. To assess the affinity of the α7 nAChR agonists of the invention on other nicotinic acetylcholine receptors, the method disclosed in WO2001/85727 can be used, i.e. to assess the affinity on human neuronal α 4β2 nAChR, a similar functional assay is carried out using a human embryonic kidney cell line stable expressing the human α4β2 subtype and to assess the activity of the compounds of the invention on the "ganglionic subtype" and the "muscle type" of nicotinic acetylcholine receptor, similar functional assays are carried out with a human embryonic kidney cell line stably expressing the human "ganglionic subtype" or a cell line endogenously expressing the human "muscle type" of nicotinic acetylcholine receptors.

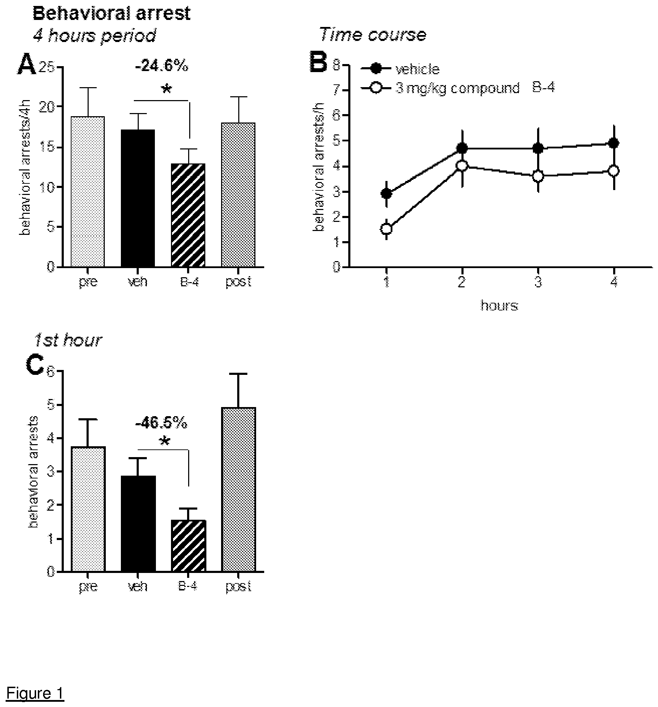

In the last 15 years much effort has been focused on developing selective α7 nAChR agonists leading to the discovery of many different chemotypes displaying said selective activity. These efforts are summarized the review from Horenstein et al (Mol Pharmacol, 2008, 74, 1496-1511, which describes no less than 9 different families of α7 nAChR agonists, in most of which selective agonists have been found. All compounds disclosed in figure 1 of said review are incorporated herein by reference. In fact, several drug candidates having an α7 nAChR agonist mode of action entered pre-clinical or even clinical testing (for review: Broad et al, Drugs of the Future, 2007, 32(2), 161-170; Romanelli et al, Expert Opin Ther Patents, 2007, 17(11), 1365-1377). Examples of such compounds - again belonging to a diversity of chemotypes - are MEM3454, MEM63908, SSR180711, GTS21, EVP6124, ABT107 and TC-5619. Further α7 nAChR agonists and their use as pharmaceuticals are known, for example, from WO2001/85727, WO2004/022556, WO2005/118535, WO2005/123732, WO2006/005608, WO2007/045478, WO2007/068476 and WO2007/068475.

Disclosed herein is
(i) a compound of formula (I) 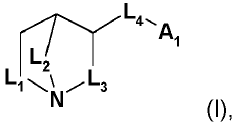 wherein
   L₁ is -CH₂-; L₂ is -CH₂-CH₂-; and L₃ is -CH₂- or -CH(CH₃)-; or
   L₁ is -CH₂-CH₂-; L₂ is -CH₂-; and L₃ is -CH₂-CH₂-;
   L₄ is a group selected from 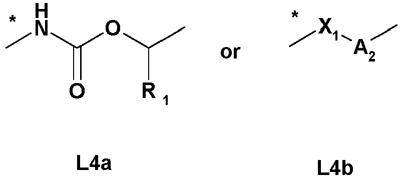
   wherein the bond marked with the asterisk is attached to the azabicycloalkyl moiety; R₁ is methyl;
   X₁ is -O- or -NH-;
   A₂ is selected from 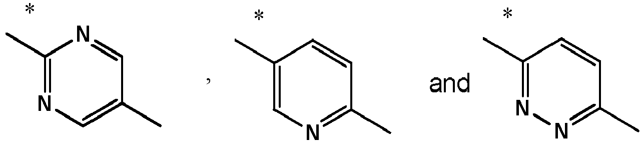
   wherein the bond marked with the asterisk is attached to X₁;
   A₁ is phenyl, indole or 1,3-dihydro-indol-2-one, which may be substituted once or more than once by R₂, each R₂ independently is C₁₋₆alkyl, C₁₋₆halogenalkyl or halogen; or
(ii) a compound selected from the group consisting of
   4-(5-phenyl-1,3,4-thiadiazol-2-yloxy)-1azatricyclo[3.3.1.1^{3,7}]decane;
   (4S)-4-(5-phenyl-1,3,4-thiadiazol-2-yloxy)-1azatricyclo[3.3.1.1^{3,7}]decane;
   4-(6-(1H-indol-5-yl)-pyridazin-3-yloxy)-1azatricyclo[3.3.1.1^{3,7}]decane;
   4-(6-(1H-indol-5-yl)-pyridin-3-yloxy)-1azatricyclo[3.3.1.1^{3,7}]decane;
   4-(5-(1H-indol-5-yl)-pyrimidin-2-yloxy)-1azatricyclo[3.3.1.1^{3,7}]decane;
   N-(1-azabicyclo[2.2.2]oct-3-yl)-1H-indazole-3-carboxamide;
   N-((3R)-1-azabicyclo[2.2.2]oct-3-yl)-1H-indazole-3-carboxamide
   N-((3S)-1-azabicyclo[2.2.2]oct-3-yl)-1H-indazole-3-carboxamide
   N-(1-azabicyclo[2.2.2]oct-3-yl)-5-(trifluoromethoxy)-1H-indazole-3-carboxamide;
   N-((3R)-1-azabicyclo[2.2.2]oct-3-yl)-5-(trifluoromethoxy)-1H-indazole-3-carboxamide;
   N-((3S)-1-azabicyclo[2.2.2]oct-3-yl)-5-(trifluoromethoxy)-1H-indazole-3-carboxamide;
   N-(2-((3-pyridinyl)methyl)-1-azabicyclo[2.2.2]oct-3-yl)benzofuran-2-carboxamide;
   (2S,3R)-N-(2-((3-pyridinyl)methyl)-1-azabicyclo[2.2.2]oct-3-yl)benzofuran-2-carboxamide;
   N-(2-((3-pyridinyl)methyl)-1-azabicyclo[2.2.2]oct-3-yl)-3,5-difluorobenzamide;
   (2S,3R)-N-(2-((3-pyridinyl)methyl)-1-azabicyclo[2.2.2]oct-3-yl)-3,5-difluorobenzamide;
   N-(2-((3-pyridinyl)methyl)-1-azabicyclo[2.2.2]oct-3-yl)-5-methylthiophene-2-carboxamide;
   (2S,3R)-N-(2-((3-pyridinyl)methyl)-1-azabicyclo[2.2.2]oct-3-yl)-5-methylthiophene-2-carboxamide;
   N-(2-((3-pyridinyl)methyl)-1-azabicyclo[2.2.2]oct-3-yl)-5-(2-pyridinyl)thiophene-2-carboxamide;
   (2S,3R)-N-(2-((3-pyridinyl)methyl)-1-azabicyclo[2.2.2]oct-3-yl)-5-(2-pyridinyl)thiophene-2-carboxamide;
   7,8,9,10-tetrahydro-6,10-methano-6H-pyrazino-(2,3-h)(3)-benzazepine;
   3-[1-(2,4-Dimethoxy-phenyl)-meth-(E)-ylidene]-3,4,5,6-tetrahydro-[2,3']bipyridinyl;
   N-methyl-1-{5-[3'H-spiro[4-azabicyclo[2.2.2]octane-2,2'-furo[2,3-b]pyridin]-5'-yl]-2-thienyl}methanamine;
   N-methyl-1-{5-[(2R)-3'H-spiro[4-azabicyclo[2.2.2]octane-2,2'-furo[2,3-b]pyridin]-5'-yl]-2-thienyl}methanamine;
   N-methyl-1-{5-[(2S)-3'H-spiro[4-azabicyclo[2.2.2]octane-2,2'-furo[2,3-b]pyridin]-5'-yl]-2-thienyl}methanamine;
   (R)-7-chloro-N-(quinuclidin-3-yl)benzo[b]thiophene-2-carboxamide;
   5-{5-[(endo)-8-azabicyclo[3.2.1]octan-3-yloxy]pyridin-2-yl}-1 H-indole;
   5-{5-[(exo)-8-azabicyclo[3.2.1]octan-3-yloxy]pyridin-2-yl}-1 H-indole;
   5-{5-[(endo)-8-methyl-8-aza-bicyclo[3.2.1]oct-3-yloxy]pyridin-2-yl}-1 H-indole;
   5-{5-[(exo)-8-methyl-8-aza-bicyclo[3.2.1]oct-3-yloxy]pyridin-2-yl}-1 H-indole;
   4-{5-[(exo)-8-methyl-8-aza-bicyclo[3.2.1]oct-3-yloxy]pyridin-2-yl}-1 H-indole;
   5-{6-[(exo)-8-methyl-8-aza-bicyclo[3.2.1]oct-3-yloxy]pyridin-3-yl}-1 H-indole;
   (2'R)-spiro-[1-azabicyclo[2.2.2]octane-3,2'(3'H)-furo[2,3-b]pyridine];
   1,4-Diaza-bicyclo[3.2.2]nonane-4-carboxylic acid 4-bromo-phenyl ester; and
   5-{6-[1-azabicyclo[2.2.2]oct-3-yloxy]pyridazin-3-yl}-1H-indole;
   in free base form or in acid addition salt form.

Unless indicated otherwise, the expressions used in this invention have the following meaning:
"Alkyl" represents a straight-chain or branched-chain alkyl group, for example, methyl, ethyl, n- or iso-propyl, n-, iso-, sec- or tert-butyl, n-pentyl, n-hexyl; C₁₋₆alkyl preferably represents a straight-chain or branched-chain C₁₋₄alkyl with particular preference given to methyl, ethyl, n-propyl, iso-propyl and tert-butyl.

The alkyl part of "halogenalkyl" and shall have the same meaning as described in the above-mentioned definition of "alkyl", especially regarding linearity and preferential size.

A substituent being substituted "once or more than once", for example as defined for A₁, is preferably substituted by one to three substituents.

Halogen is generally fluorine, chlorine, bromine or iodine; preferably fluorine, chlorine or bromine. Halogenalkyl groups preferably have a chain length of 1 to 4 carbon atoms and are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,2-trichloroethyl, 1,1,2,2-tetrafluoroethyl, 2,2,3,3-tetrafluoropropyl, 2,2,3,3,3-pentafluoropropyl or 2,2,3,4,4,4-hexafluorobutyl; preferably -CF₃, -CHF₂, -CH₂F, -CHF-CH₃, -CF₂CH₃, or -CH₂CF₃.

In the context of the invention, the definition of A₁ or A₃ as a "five- to ten-membered monocyclic or fused polycyclic aromatic ring system" encompasses a C₆- or C₁₀-aromatic

The acid addition salt forms of the α7 nAChR agonists of the invention are preferably pharmaceutically acceptable salt forms. Such salts are known in the field (e.g. S.M. Berge, et al, "Pharmaceutical Salts", J. Pharm. Sd., 1977, 66:1-19; and "Handbook of Pharmaceutical Salts, Properties, Selection, and Use", Stahl, RH., Wermuth, C.G., Eds.; Wiley-VCH and VHCA: Zurich, 2002). A "pharmaceutically acceptable salt form" is intended to mean a salt form that is not toxic, biologically intolerable, or otherwise biologically undesirable.

On account of asymmetrical carbon atom(s) that may be present in the compounds of formula (I), unless stated otherwise, the compounds may exist in optically active form or in form of mixtures of optical isomers, e.g. in form of racemic mixtures or diastereomeric mixtures. Unless stated otherwise, all optical isomers and their mixtures, including racemic mixtures, are part of the present invention.

The α7 nAChR agonist of the invention is a compound B-1: (R)-3-(6-p-tolyl-pyridin-3-yloxy)-1-aza-bicyclo[2.2.2]octane; in free base form or in acid addition salt form.

In one embodiment, the α7 nAChR agonist of the invention is a compound B-1 in free base form or in acid addition salt form. In another embodiment, the α7-nAChR agonist of the invention is compound B-1, which is in fumarate salt form. In yet another embodiment, the α7-nAChR agonist of the invention is the mono-fumarate salt of compound B-1.

The compounds of formula (I) (e.g. compound B-1) and their manufacture are known from WO2001/85727, WO2004/022556, WO2005/123732, WO2006/005608, WO2007/045478, WO2007/068476 and WO2007/068475, or can be prepared analogously to said references.

Uses in therapy:
"Fatigue" as used herein refers to a prolonged state in a subject which is characterized by physical and/or mental weakness and is caused by a disorder.

In one embodiment, fatigue is present in a subject for more than one day.

In one embodiment, fatigue is present in a subject for more than one week.

In one embodiment, fatigue is present in a subject for more than one month.

Disorders causing fatigue are listed above, and may particularly be, e.g. chronic fatigue syndrome (CFS); multiple sclerosis (MS); or infectious diseases, e.g. HIV infection.

Fatigue has an onset (i.e. is not lifelong) and at least some of the following criteria should be present: (i) a significant reduction of previous activity levels; (ii) impaired memory or concentration; (iii) post-exertional malaise, where physical or mental exertions bring on extreme, prolonged exhaustion and sickness; (iv) unrefreshing sleep; (v) muscle pain; (vi) pain in muscle joints; (vii) headaches of new kind of severity; (viii) frequent or recurring sore throat; or (ix) cervical or axillary tender lymph nodes.

In one embodiment, at least four of the above criteria are present in fatigue.

In one embodiment, at least five of the above criteria are present in fatigue.

In one embodiment, at least six of the above criteria are present in fatigue.

Fatigue can be e.g. chronic fatigue syndrome (CFS), fatigue associated with MS or fatigue associated with an infectious disease, e.g. HIV infection.

The term "subject" as used herein refers to a human being, especially to a patient suffering from fatigue, e.g. CFS or fatigue associated with MS.

The term "treatment" as used herein refers to any type of treatment that imparts a benefit to a subject, especially a patient, suffering from fatigue, including a reduction of one or more characteristics associated with fatigue or prevention/delay of the onset/progression of fatigue (e.g. prophylactic treatment).

The term "therapeutically effective amount" as used herein typically refers to a drug amount which, when administered to a subject, is sufficient to provide a therapeutic benefit, e.g. is sufficient for treating, ameliorating, preventing or delaying the progression of fatigue (e.g. the amount provides a amelioration of the characteristics associated with fatigue, e.g. it leads to an increase of activity levels of the subject).

Treatment may comprise a reduction in the characteristics associated with fatigue, including for example, although not limited to, a reduction in the degree of physical and/or mental weakness; a reduction in the number of fatigue attacks; an improvement in the well-being of the subject; an improvement in the ability to carry out normal tasks; an improved ability to drive cars and operate machines, and/or an increased period of time between fatigue attacks.

One aspect of the treatment of fatigue is that said treatment should be efficacious and have a minimal adverse effect on the patient, e.g. the drug used should have a high level of cardiac safety.

Although there are currently no drugs approved for fatigue (e.g. fatigue associated with MS or CFS), certain drugs approved for other conditions are sometimes prescribed. Examples include: amantadine, modafinil and Prokarin.

Amantadine is approved for Parkinson's disease, as well as some viral infections. Research regarding its use in treating fatigue associated with MS is not conclusive. Side effects can include insomnia and vivid dreams.

Modafinil is used to treat narcolepsy, a sleep disorder which causes people to sleep excessively during the day. There have been several small studies looking at modafinil to treat fatigue associated with MS, but they have had conflicting results and have not proved the benefits of taking modafinil. Side effects can include insomnia and headaches. Prokarin is a skin patch that contains caffeine and histamine. Reported side effects are rashes at the side of the patch.

Antidepressants are also used as many people who have chronic fatigue syndrome are also depressed. Low doses of some antidepressants can help improve sleep and relieve pain, but have limited overall efficacy.

Highly relevant would be e.g. an agent that can be used to treat fatigue without producing the side effects of the above treatment regiments.

In the case of prophylactic treatment, the α7 nAChR agonists of the invention may be used to delay or prevent the onset of fatigue, e.g. in MS patients.

For the above-mentioned treatment methods the appropriate dosage will vary depending upon, for example, the compound employed, the host, the mode of administration and the nature and severity of the condition/symptom being treated. However, in general, satisfactory results in animals are indicated to be obtained at a daily dosage of from 0.1 to 100 mg/kg body weight, preferably from 1 to 50 mg/kg body weight, e.g. 10 mg/kg. In larger mammals, for example humans, an indicated daily dosage is in the range from 0.5 to 500 mg, preferably from 1 to 100 mg, most preferably from 2 to 75 mg, e.g. 50 mg of an α7 nAChR agonist of the invention conveniently administered, for example, in divided doses up to four times a day.

In one embodiment, the daily dosage of the α7 nAChR agonist of the invention is from 1 to 100 mg.

In one embodiment, the daily dosage of the α7 nAChR agonist of the invention is from 2 to 75 mg.

In one embodiment, the daily dosage of the α7 nAChR agonist of the invention is about 50 mg.

### Chronic fatigue syndrome (CFS):

Recent studies have reported between 7 and 3,000 cases of CFS for every 100,000 adults (N Afari and D Buchwald, 2003, "Chronic fatigue syndrome: a review". Am J Psychiatr, 160(2): 221-36). The wide variance of the prevalence estimates may be due to the different definitions of CFS in use, the settings in which patients were selected, and the methodology used to exclude study participants with possible alternative diagnoses (G Ranjith, 2005, "Epidemiology of chronic fatigue syndrome". Occup Med (Lond) 55 (1): 13-29). The Centers for Disease Control report that more than 1 million Americans have CFS and approximately 80% of the cases are undiagnosed ("Chronic Fatigue Syndrome Basic Facts", Centers for Disease Control and Prevention. May 9, 2006). Approximately 250,000 people in the UK are affected with the illness according to the National Health Service ("Chronic fatigue syndrome"; The National Health Service, 2009-06-29).

Quality of life is severely disrupted by CFS. CFS typically has a sudden onset usually accompanied by a flu-like illness and a significant proportion of cases begin within several months of severe adverse stress. CFS may also be caused by viral or non-viral pathogen infection. Most commonly used diagnostic criteria were published by the United States Centers for Disease Control and Prevention (CDC). CDC recommends the following three criteria to be fulfilled:
1. A new onset (not lifelong) of severe fatigue for six consecutive months or greater duration which is unrelated to exertion, is not substantially relieved by rest, and is not a result of other medical conditions.
2. The fatigue causes a significant reduction of previous activity levels.
3. Four or more of the following symptoms that last six months or longer:
   - impaired memory or concentration;
   - post-exertional malaise, where physical or mental exertions bring on "extreme, prolonged exhaustion and sickness";
   - unrefreshing sleep;
   - muscle pain (myalgia);
   - pain in multiple joints (arthralgia);
   - headaches of a new kind or greater severity;
   - sore throat, frequent or recurring; or
   - tender lymph nodes (cervical or axillary).
CFS may be persistent or relapsing.

### Fatigue associated with multiple sclerosis:

Up to 75% of multiple sclerosis (MS) patients suffer at some stage during their disease from fatigue associated with multiple sclerosis (Henze et al, European Neurology, 2006, 56, 78-105).

Fatigue associated with MS may be caused by sleep deprivation (e.g. due to bladder dysfunction or nocturnal muscle spasms producing night-time awakenings); depression; or general disabilities (i.e. the patient needs considerable effort to accomplish daily tasks). Additionally fatigue can be associated with MS but cannot be directly correlated with sleep deprivation, depression or general disability; this type of fatigue associated with MS is also referred to as "lassitude".

Fatigue associated with MS typically has the following characteristics: it generally occurs on a daily basis, may occur early in the morning, even after a restful night's sleep, tends to worsen as the day progresses, tends to be aggrevated by heat and/or humitity, comes on easily and suddenly, is generally more severe than fatigue not associated with MS and is more likely to interfere with daily responsibilities.

### Pharmaceutical compositions:

For use according to the invention, the α7 nAChR agonist of the invention may be administered as single active agent or in combination with other active agents, in any usual manner, e.g. orally, for example in the form of tablets or capsules, parenterally, for example in the form of injection solutions or suspensions, or transdermally, for example in the form of a patch.

In one embodiment, the manner of administration is oral administration, for example in the form of tablets or capsules.

Moreover, the present invention provides a pharmaceutical composition comprising an α7 nAChR agonist of the invention in association with at least one pharmaceutical carrier or diluent for the treatment, amelioration, prevention or delay of progression fatigue. Such compositions may be manufactured in conventional manner. Unit dosage forms may contain, for example, from about 2.5 to about 25 mg of one or more of the α7 nAChR agonist of the invention.

The pharmaceutical compositions according to the invention are compositions for enteral, such as nasal, rectal or oral; parenteral, such as intramuscular or intravenous; or transdermal (e.g. by a patch) administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragees, tablets or capsules.

The pharmaceutical compositions of the present invention are prepared in a manner known per se, for example by means of conventional dissolving, lyophilizing, mixing, granulating or confectioning processes. Such processes are exemplified in WO 2005/079802, WO 2003/047581, WO 2004/000316, WO 2005/044265, WO 2005/044266, WO 2005/044267, WO 2006/114262 and WO 2007/071358.

Compositions for transdermal are described in Remington's Pharmaceutical Sciences 16th Edition Mack; Sucker, Fuchs and Spieser, Pharmazeutische Technologie, 1st Edition, Springer.

The following are further embodiments of the invention.

Further embodiments of the invention:
Embodiment 1-1: An α7 nAChR agonist of the invention for use in the treatment, amelioration, prevention or delay of progression of fatigue; wherein said α7 nAChR agonist is a compound selected from compound B-1 in free base form or in acid addition salt form.
Embodiment 1-2. The α7 nAChR agonist as defined in embodiment 1-1, wherein the fatigue is fatigue associated with multiple sclerosis.
Embodiment 1-3. The α7 nAChR agonist as defined in embodiment 1-2, wherein the fatigue is fatigue associated with multiple sclerosis and is (i) caused by sleep deprivation, depression or general disabilities or (ii) lassitude.
Embodiment 1-4. The α7 nAChR agonist as defined in embodiment 1-1, wherein the fatigue is chronic fatigue syndrome.
Embodiment 1-5. The α7 nAChR agonist as defined in embodiment 1-1, wherein the fatigue is fatigue associated with an infectious disease.
Embodiment 1-6. The α7 nAChR agonist as defined in embodiment 1-5, wherein the fatigue is fatigue associated with HIV infection.
Embodiment 1-7. The α7 nAChR agonist as defined in any of embodiments 1-1 to 1-6, wherein the daily dosage of said agonist is from 1 to 100 mg.

The following non-limiting examples are illustrative of the disclosure.

### 1. Formulation Examples

### 1.1 Hard Capsules

Hard gelatin capsules, each comprising as active ingredient 0.5, 5 or 25 mg of the mono-fumarate of compound B-1, i.e. of (R)-3-(6-(4-methylphenyl)-pyridin-3-yloxy)-1-aza-bicyclo[2.2.2]octane, can be prepared as follows:

| Ingredient for capsule fill | % (w/w) for 0.5 mg capsules | % (w/w) for 5 mg capsules | % (w/w) for 25 mg capsules |
|---|---|---|---|
| Mono-fumarate of (R)-3-(6-(4-methylphenyl)-pyridin-3-yloxy)-1-aza-bicyclo[2.2.2]octane | 0.46 | 4.65 | 23.23 |
| Lactose monohydrate | 65.24 | 61.05 | 42.47 |
| Microcrystalline cellulose | 25.00 | 25.00 | 25.00 |
| Hypromellose | 2.50 | 2.50 | 2.50 |
| Sodium croscarmellose | 6.00 | 6.00 | 6.00 |
| Colloidal silicon dioxide | 0.30 | 0.30 | 0.30 |
| Magnesium stearate | 0.50 | 0.50 | 0.50 |
| Purified water* | q.s. | q.s. | q.s. |

| | | | |
|---|---|---|---|
| * removed during processing | | | |

Preparation process: Mono-fumarate of (R)-3-(6-(4-methylphenyl)-pyridin-3-yloxy)-1-aza-bicyclo[2.2.2]octane, lactose monohydrate, microcrystalline cellulose, a portion of sodium croscarmellose and hypromellose are dry mixed in a high shear mixer bowl, and granulating fluid (purified water) added. Once the granulation is complete, the wet granules are dried in a fluid bed drier and the dry granules are milled. The remaining sodium croscarmellose and colloidal silicon dioxide are passed through a suitable sieve and added to the dried granular material and blended in a suitable blending shell. This is achieved by co-sieving the sodium croscarmellose and the colloidal silicon dioxide with a portion of the milled granules through a suitable sieve into the blending shell. Similarly, the required amount of sieved magnesium stearate is added to the bulk granule and then mixed in the same blending shell. This final blend is encapsulated into capsules using automated equipment. Weigth ratio of capsule fill to empty capsule shells is 2 : 1.

### 1.2: Tablets

### Example 1.2.1: Film-coated tablet

Film-coated tablets containing e.g. 0.5 mg of the mono-fumarate of (R)-3-(6-(4-methylphenyl)-pyridin-3-yloxy)-1-aza-bicyclo[2.2.2]octane may be prepared as follows:

### Preparation of pre-mix:

Weigh-in mono-fumarate of (R)-3-(6-(4-methylphenyl)-pyridin-3-yloxy)-1-aza-bicyclo[2.2.2]octane (e.g. approx. 0.7%) and maize starch (e.g. approx. 13%), mix in a tumble blender (approx 100-300 rotations), pass through a sieve of approx. 0.25-1.0 mm mesh-size. Mix in a tumble blender (approx. 100-300 rotations).

### Preparation of final blend:

To above pre-mix add microcrystalline cellulose (e.g. approx. 25%), sprayed lactose (e.g. approx. 68%), sodium-carboxymethylcellulose XL (e.g. approx. 2%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 1.5%) through a handsieve at approx. 0.5-1.0 mm mesh-size and mix in a tumble blender (approx. 30-150 rotations).

### Compression:

On a rotary press compress the above final blend to cores of approx. 100mg, using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example 1.2.2: Bilayer film-coated tablet

Bilayer film-coated tablets containing e.g. 2.5 mg of the mono-fumarate of (R)-3-(6-(4-methylphenyl)-pyridin-3-yloxy)-1-aza-bicyclo[2.2.2]octane may be prepared as follows:

### Final active blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the Na-stearyl-fumarate (e.g. approx. 3%) through a handsieve at approx. 0.5-10mm and mix in a tumble blender (approx 30-150 rotations).

### Final placebo blend:

Weigh-in microcrystalline cellulose (e.g. approx. 26%), sprayed lactose (e.g. approx. 69%), sodium-carboxymethylcellulose XL (e.g. approx. 1.9%) and Aerosil (e.g. approx. 0.5%) and mix in a tumble blender (approx 100-300 rotations). Pass this mixture through a sieve of approx. 0.5-1.0 mm mesh-size and mix again (approx 100-300 rotations).

Add the sodium-stearyl-fumarate (e.g. approx. 3%) through a handsieve at approx. 0.5-1.0 mm and mix in a tumble blender (approx 30-150 rotations).

### Compression:

On a rotary press compress the above final blends to a bilayer tablet-core of approx. 100mg with one placebo layer (approx. 77.5mg) and one active layer (approx. 22.5mg), using the dosage specific tooling (e.g. approx. 6mm, round, curved).

### Coating:

Prepare a suspension in water with basic coating premixes black, red, yellow and/or white. Coat the above obtained cores in a perforated coating pan, and dry.

### Example 1.2.3: Film-coated tablet

Film-coated tablets containing e.g. 50 mg of the mono-fumarate of (R)-3-(6-(4-methylphenyl)-pyridin-3-yloxy)-1-aza-bicyclo[2.2.2]octane may be prepared as follows:

### Final blend:

Weigh-in mono-fumarate of (R)-3-(6-(4-methylphenyl)-pyridin-3-yloxy)-1-aza-bicyclo[2.2.2]octane coarse (e.g. approx. 15.5%), microcrystalline cellulose (e.g. approx. 25%), sprayed lactose (e.g. approx. 53%), sodium-carboxymethylcellulose XL (e.g. approx. 3%) and Aerosil (e.g. approx. 0.5%) and mix in a tumble blender (approx. 100-300 rotations). Pass this mixture through a sieve of approx. 0.5-1.0 mm mesh-size and mix again (approx. 100-300 rotations).

Add the sodium-stearyl-fumarate (e.g. approx. 3%) through a handsieve at approx. 0.5-10mm and mix in a tumble blender (approx. 30-150 rotations).

### Compression:

Compress the above final blend on a rotary press to cores, using the dosage specific tooling (e.g. approx. 15*5.9mm, round, curved).

### Coating:

Prepare a suspension in water with basic coating premixes black, red, yellow and/or white. Coat the above obtained cores in a perforated coating pan, and dry.

The usefulness of the α7 nAChR agonists of the invention in the treatment of fatigue can be confirmed in a range of standard tests including those indicated below.

### 2. Preclinical testing

### 2.1. In-vitro Tests: Selectivity of Compounds A-1 and B-1

### A-1: (S)-(1-aza-bicyclo[2.2.2]oct-3-yl)-carbamic acid (S)-1-(2-fluoro-phenyl)-ethyl ester;

Based on the activity/selectivity data shown below it is concluded that said compounds are selective agonists at the α7-nAChR.

Assays: To assess α7-nAChR activity, a functional assay was employed using GH3 cells that recombinantly expressed human α7-nAChR. 40000 cells per well were seeded 48 h prior to the experiment on black 96-well plates (Costar) and incubated at 37°C in a humidified atmosphere (5 % CO₂/95 % air). On the day of the experiment, medium was removed by flicking the plates and replaced with 0.1 ml growth medium containing 0.002 mM Fluo-4, (Molecular Probes) in the presence of 2.5 mM probenecid (Sigma). The cells were incubated at 37°C in a humidified atmosphere (5 % CO2/95 % air) for 1 h. Plates were flicked to remove excess of Fluo-4, washed twice with Hepes-buffered salt solution (HBSS, in mM: NaCl 130, KCl 5.4, CaCl₂ 2, MgSO₄ 0.8, NaH₂PO₄ 0.9, glucose 25, Hepes 20, pH 7.4; HBS) and refilled with 0.1 ml of HBS containing antagonist when appropriate. The incubation in the presence of the antagonist lasted 3-5 minutes. Plates were placed in the cell plate stage of a FLIPR device (fluorimetric imaging plate reader, Molecular Devices, Sunnyvale, CA, USA). After recording of the baseline (laser: excitation 488 nm at 1 W, CCD camera opening of 0.4 seconds) the agonists (0.05 ml) were added to the cell plate using the FLIPR 96-tip pipettor while simultaneously recording the fluorescence. Calcium kinetic data were normalized to the maximal fitted response induced by epibatidine, which is a full agonist at α7-nAChR. Four parameter Hill equations were fitted to the concentration-response. Values of Emax (maximal effect in % compared to the epibatidine response) and EC₅₀ (concentration producing half the maximal effect in µM) were derived from this fit. Assay described in: D Feuerbach et al, Neuropharmacology (2005), 48, 215-227.

To assess the activities of the compounds on the other receptors (i.e. α1β1γδ; α4β2; α3β4 and 5-HT₃), similar functional assays were carried out.

| | Compound B-1 | Compound A-1 |
|---|---|---|
| α7 EC₅₀ (nM) ± SEM, n | 39 ± 4.7, 18 | 100 ± 7.9,17 |
| α7 Eₘₐₓ ± SEM, n | 73 ± 4.1%, 18 | 85 ± 5%, 17 |
| α1β1γδ IC₅₀(nM) ± SEM, n | 10715 ± 1376, 6 | 159222 ± 4306, 6 |
| α4β2 IC₅₀ (nM) ± SEM, n | 3981 ± 770, 6 | 25119 ± 4794, 5 |
| α3β4 IC₅₀ (nM)± SEM, n | 5248 ± 609, 5 | 23097± 4123, 6 |
| 5-HT₃ IC₅₀ (nM) ± SEM, n | 19054 ± 3811, 6 | 22324 ± 5869, 2 |

### 2.2. In-vivo pharmacology

### 2.2.1 Orexin-deficiency model:

### a) Animals

Male transgenic mice with a knockout of the orexin peptide gene are used. Orexin-deficient mice express a phenotype with cataplexy events (observed as 'behavioral arrests') and a disrupted sleep pattern including fragmented sleep and sleepiness in their active period.

### b) Assessment of behavior

The animals are observed and videotaped via infrared cameras during the first hours of the lights-off period which is the active phase of nocturnal rodents like mice. At this time, orexin-deficient mice show the most 'behavioral arrests'. The number of these events can be furthermore increased by presenting a new environment. For this purpose, new saw dust, a running wheel, marbles and tunnels are placed in the test environment. The number of 'behavioral arrests' are offline counted by the experimenter for each hour of recording. Behavioral arrests were defined by phases of total inactivity outside the next box, which last longer than 10 second, preceded and followed by robust locomotor activity (see also Scammell et al., 2009, Sleep 32(1): 111-116). Some of the animals have temporal skull EEG electrodes implanted which are connected to transmitters. The EEG is recorded via a receiver and stored on a PC for further analysis. EEG is used to assess and to quantify sleep behavior and vigilance stage.

### c) Protocol

A cross-over design is used because of the high inter- and intra-individual variability of the behaviors to be observed. Furthermore, to avoid habituation to the test environment, the animals are only tested once per week in the test environment. During the remaining days, the animals are in their homecage.

For each experimental session, 3-4 mice are put into the test environment 3 hours before lights-off. The first experimental session (week 1) is used to get a baseline (without treatment). The second and third sessions (weeks 2+3) are used for treatment (vehicle & compound, cross-over design, administration 5 minutes before lights-off). The fourth and last experimental session is again without treatment to evaluate a post-treatment baseline.

### d) Results

### B-4: (2S,3R)-3-[6-(1H-indol-5-yl)-pyridazin-3-yloxy]-2-methyl-1-aza-bicyclo[2.2.2]octane;

Figure 1 depicts the effects of 3 mg/kg compound B-4, orally administered directly before lights-off, on the number of narcoleptic episodes (behavioral arrests) in orexin-deficient mice. Compound B-4 decreased the number of narcoleptic episodes during the whole 4 hours observation period by ca. 25 % (Paired t-test: t₁₄ = 2.48, p = 0.03; Figure 1A+B). The reduction was more pronounced during the first hour (ca. 47%; t₁₄ = 2.32, p = 0.04; Figure 1C).

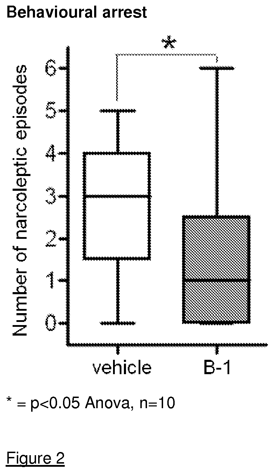

Figure 2 depicts the effects of 10 mg/kg compound B-1, orally administered directly before lights-off, on the number of narcoleptic episodes (behavioral arrests) in orexin-deficient mice. Compound B-1 significantly decreased the number of narcoleptic episodes during the first hour of the observation period by ca. 66 % (p<0.05 Anova).

### 2.2.2 EEG data

### a) Animals

Animals have temporal skull EEG electrodes implanted which are connected to transmitters.

Animals were group-housed (2 - 4) per cage at a constant temperature of 22 ± 1 °C, on a 12:12 light-dark schedule. The EEG is recorded via a receiver and stored on a PC for further analysis. EEG is used to assess and to quantify sleep behavior and vigilance stage.

### b) Protocol

Animals were given a 1-day period of acclimatization in the recording cages with recording cables connected. On the 1st day of the experiment, EEG recordings were made continuously during a 22-hours period beginning at 11 A.M., 15 min after vehicle administration. On the second day of the experiment, EEG recordings were carried out in the same way, but 15 minutes after drug administration. In this way, each animal served as its own control. 10 animals were recorded from simultaneously in each experiment. Automatic evaluation of the nycthemeral cycle (REM sleep, classical sleep, wakefulness and an awake non-vigilant state termed "drowsiness") was performed as described (Vigouret et al., 1978 Pharmacology 16 Suppl 1:156-73). In the present context, "quiet wake" and "active wake", describe conscious animals in a resting and an active state, respectively, while "REM" and "NREM" represent sleep states.

### c) Results

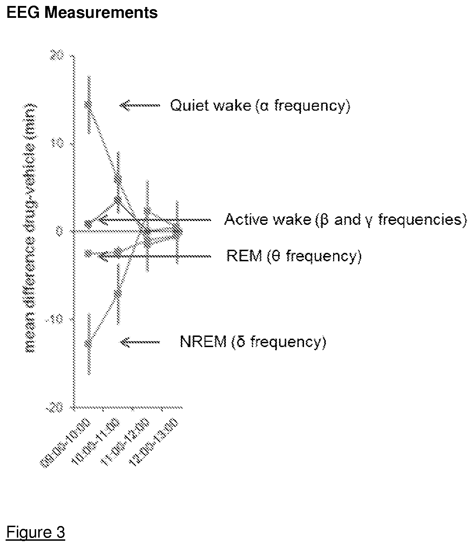

Figure 3 depicts the effects of 30 mg/kg compound B-1, orally administered 6 h after lights on (9:00) in mice (n=10) on quiet wake (α frequency, 9.5-12.5 Hz), active wake (β frequency, 12.5-30.5 Hz; and γ frequency, >30.5 Hz), REM sleep (θ frequency, 4.5-9.5 Hz) and NREM sleep (δ frequency, <4.5 Hz). Assessment period shown is 3 hours. Compound B-1 causes a decline of NREM sleep and increases the time spent in quiet wake. The numerical assessment of figure 3 is given in table 1.

**Table 1:**

| | Mean difference drug-vehicle (min) assessed at time point | | | |
|---|---|---|---|---|
| | 9:30 | 10:30 | 11:30 | 12:30 |
| Quiet wake (α frequency) | 14.3 | 6.1 | -1.8 | -0.9 |
| Active wake (β and γ frequencies) | 0.9 | 3.6 | 0.1 | 0.3 |
| REM (θ frequency) | -2.2 | -2.1 | -0.9 | -0.2 |
| NREM (δ frequency) | -12.4 | -7.1 | 2.1 | -0.1 |

### 2.2.3 telemethyl histamine

Histamine (HA) is released all over the brain by tuberomammillary nucleus neurons. Evidence indicates that histaminergic neurons have a major role in wakefulness control. HA is metabolized by histamine-N-methyltransferase to tele-methylhistamine (tMHA) in some tissues, including brain.

### a) Protocol

Mice (n=8) were treated during the first hour of the lights-on period and sacrificed 30 min later. Brain tissue was collected and subjected to LC-MS analysis (Croyal et al. Analytical Biochemistry, 2011, 409:28-36) to determine t-MHA levels.

### b) Results

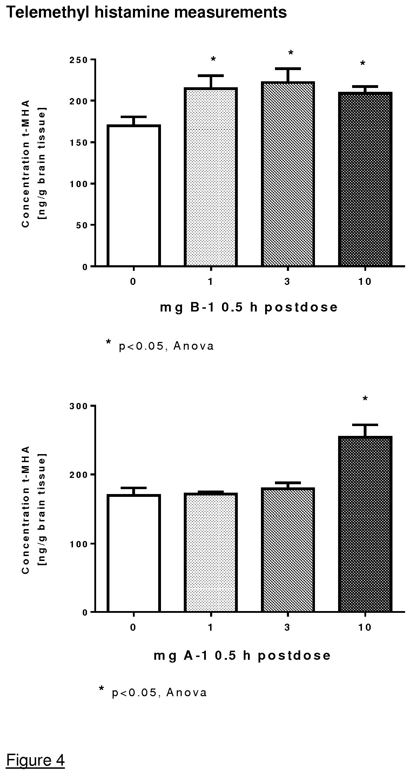

Figure 4 depicts the effects of different doses of compound B1 and A-1, orally administered to mice, 30 minutes after administration on brain tMHA levels. tMHA levels are significantly elevated for compound B-1 at all doses tested. For compound A-1, a significant elevation is seen for the 10 mg/kg dose.

### 3. Clinical Testing: Improvement Trials

Clinical testing of the α7 nAChR agonist of the invention may be conducted, for example, in one of the following study designs. The skilled physician may look at a number of aspects of patient behaviors and abilities. He will realize that such studies are considered as guidelines and the certain aspects of the studies may be modified and redefined depending on the circumstance and environment, for example.

### 3.1 Trial A: Normal Patient Population

A patient population, with a normal control is dosed once a day for a week or longer tested. The test is designed to allow for improvement, i.e. that there is a measurable parameter increase of the impaired function. The patients are tested at the beginning and at the end of the dosage period and the results are compared and analyzed.

### 3.2 Trial B: Deficit population

A patient population with fatigue: e.g. fatigue associated with MS is dosed once a day for 12 weeks or longer and tested. The test is designed to allow for improvement, i.e. that there is a measurable parameter increase of the impaired domain. Examples of those tests include cognitive testing, the fatigue scale for motor and cognitive functions (FMSC), Paced Auditory Serial Addition Test (PASAT), change in fatigue as measured on a visual analog scale (VAS), Fatigue Severity Scale (FSS), Neurological status as measured with the Expanded Disability Status Scale (EDSS) and Quality Of Life among others

The patients are tested at the beginning and at the end of the dosage period and the results are compared and analyzed.

### 3.3 Considerations for designing a trial

- When designing a trial, the skilled person will appreciate the need to protect both against floor and ceiling effects. In other words, the study design should allow the specific domain to be measurably raised or lowered.
- Conditions that artificially impair a function are one way to test enhancement of that function. Such conditions are, for example, sleep deprivation or pharmacological challenges.
- Placebo control is required for all trials.

### Description of Figures:

Figure 1: Behavioral arrest, compound B-4
Figure 2: Behavioral arrest, compound B-1
Figure 3: EEG Measurements, compound B-1
Figure 4: Telemethyl histamine measurements, compounds B-1 and A-1

A further aspect of the invention relates to a pharmaceutical composition comprising the α7 nAChR agonist of the invention for the treatment, amelioration, prevention or delay of progression of fatigue.

A further aspect of the invention relates to a kit comprising an α7 nAChR agonist of the invention and instructions for using the agonist in the treatment, amelioration, prevention or delay of progression of fatigue in a subject in need of such treatment.

## Claims

1. An alpha 7 nicotinic acetylcholine receptor agonist for use in the treatment, amelioration, prevention or delay of progression of fatigue caused by a disorder; wherein said alpha 7 nicotinic acetylcholine receptor agonist is (R)-3-(6-p-tolyl-pyridin-3-yloxy)-1-aza-bicyclo[2.2.2]octane in free base form or in acid addition salt form.

2. The alpha 7 nicotinic acetylcholine receptor agonist for use as defined in claim 1, wherein the fatigue is fatigue associated with multiple sclerosis.

3. The alpha 7 nicotinic acetylcholine receptor agonist for use as defined in claim 2, wherein the fatigue is fatigue associated with multiple sclerosis and is (i) caused by sleep deprivation, depression or general disabilities or (ii) lassitude.

4. The alpha 7 nicotinic acetylcholine receptor agonist for use as defined in claim 1, wherein the fatigue is chronic fatigue syndrome.

5. The alpha 7 nicotinic acetylcholine receptor agonist for use as defined in claim 1, wherein the fatigue is fatigue associated with an infectious disease.

6. The alpha 7 nicotinic acetylcholine receptor agonist for use as defined in claim 5, wherein the fatigue is fatigue associated with HIV infection.

7. The alpha 7 nicotinic acetylcholine receptor agonist for use as defined in any of claims 1 to 6, wherein the daily dosage of said agonist is from 1 to 100 mg.

8. A pharmaceutical composition comprising (R)-3-(6-p-tolyl-pyridin-3-yloxy)-1-aza-bicyclo[2.2.2]octane in free base form or in acid addition salt form, for use in the treatment, amelioration, prevention or delay of progression of fatigue caused by a disorder.

9. The pharmaceutical composition for use according to claim 8, wherein the amount of (R)-3-(6-p-tolyl-pyridin-3-yloxy)-1-aza-bicyclo[2.2.2]octane is from 1 to 100 mg.

## Patentansprüche

1. Agonist des alpha-7-nikotinischen Acetylcholinrezeptors zur Verwendung bei der Behandlung, Linderung, Prävention oder der Verzögerung des Fortschreitens von durch eine Erkrankung verursachter Erschöpfung, wobei es sich bei dem Agonisten des alpha-7-nikotinischen Acetylcholinrezeptors um (R)-3-(6-p-Tolylpyridin-3-yloxy)-1-azabicyclo[2.2.2]octan in Form der freien Base oder in Säureadditionssalzform handelt.

2. Agonist des alpha-7-nikotinischen Acetylcholinrezeptors zur wie in Anspruch 1 definierten Verwendung, wobei es sich bei der Erschöpfung um mit multipler Sklerose assoziierte Erschöpfung handelt.

3. Agonist des alpha-7-nikotinischen Acetylcholinrezeptors zur wie in Anspruch 2 definierten Verwendung, wobei es sich bei der Erschöpfung um mit multipler Sklerose assoziierte Erschöpfung handelt, die (i) durch Schlafentzug, Depression oder allgemeine Behinderungen verursacht wird oder (ii) Abgeschlagenheit ist.

4. Agonist des alpha-7-nikotinischen Acetylcholinrezeptors zur wie in Anspruch 1 definierten Verwendung, wobei es sich bei der Erschöpfung um chronisches Erschöpfungssyndrom handelt.

5. Agonist des alpha-7-nikotinischen Acetylcholinrezeptors zur wie in Anspruch 1 definierten Verwendung, wobei es sich bei der Erschöpfung um eine mit einer Infektionskrankheit assoziierte Erschöpfung handelt.

6. Agonist des alpha-7-nikotinischen Acetylcholinrezeptors zur wie in Anspruch 5 definierten Verwendung, wobei es sich bei der Erschöpfung um eine mit einer HIV-Infektion assoziierte Erschöpfung handelt.

7. Agonist des alpha-7-nikotinischen Acetylcholinrezeptors zur wie in einem der Ansprüche 1 bis 6 definierten Verwendung, wobei die Tagesdosis des Agonisten 1 bis 100 mg beträgt.

8. Pharmazeutische Zusammensetzung, umfassend (R)-3-(6-p-Tolylpyridin-3-yloxy)-1-azabicyclo[2.2.2]octan in Form der freien Base oder in Säureadditionssalzform, zur Verwendung bei der Behandlung, Linderung, Prävention oder der Verzögerung des Fortschreitens von durch eine Erkrankung verursachter Erschöpfung.

9. Pharmazeutische Zusammensetzung zur Verwendung nach Anspruch 8, wobei die Menge an (R)-3-(6-p-Tolylpyridin-3-yloxy)-1-azabicyclo[2.2.2]octan 1 bis 100 mg beträgt.

## Revendications

1. Agoniste du récepteur nicotinique de l'acétylcholine alpha 7 pour une utilisation dans le traitement, l'amélioration, la prévention ou le retardement de la progression de la fatigue causée par un trouble ; ledit agoniste du récepteur nicotinique de l'acétylcholine alpha 7 étant le (R)-3-(6-p-tolyl-pyridin-3-yloxy)-1-aza-bicyclo[2.2.2]octane sous forme de base libre ou sous forme de sel d'addition d'acide.

2. Agoniste du récepteur nicotinique de l'acétylcholine alpha 7 pour une utilisation telle que définie dans la revendication 1, la fatigue étant une fatigue associée à une sclérose en plaques.

3. Agoniste du récepteur nicotinique de l'acétylcholine alpha 7 pour une utilisation telle que définie dans la revendication 2, la fatigue étant une fatigue associée à une sclérose en plaques et étant (i) causée par une privation de sommeil, une dépression ou des handicaps généraux ou (ii) une lassitude.

4. Agoniste du récepteur nicotinique de l'acétylcholine alpha 7 pour une utilisation telle que définie dans la revendication 1, la fatigue étant un syndrome de fatigue chronique.

5. Agoniste du récepteur nicotinique de l'acétylcholine alpha 7 pour une utilisation telle que définie dans la revendication 1, la fatigue étant une fatigue associée à une maladie infectieuse.

6. Agoniste du récepteur nicotinique de l'acétylcholine alpha 7 pour une utilisation telle que définie dans la revendication 5, la fatigue étant une fatigue associée à une infection par le VIH.

7. Agoniste du récepteur nicotinique de l'acétylcholine alpha 7 pour une utilisation telle que définie dans l'une quelconque des revendications 1 à 6, le dosage quotidien dudit agoniste étant de 1 à 100 mg.

8. Composition pharmaceutique comprenant du (R)-3-(6-p-tolyl-pyridin-3-yloxy)-1-aza-bicyclo[2.2.2]octane sous forme de base libre ou sous forme de sel d'addition d'acide, pour une utilisation dans le traitement, l'amélioration, la prévention ou le retardement de la progression de la fatigue causée par un trouble.

9. Composition pharmaceutique pour une utilisation selon la revendication 8, la quantité de (R)-3-(6-p-tolyl-pyridin-3-yloxy)-1-aza-bicyclo[2.2.2]octane étant de 1 à 100 mg.
